# EUROPEAN PATENT APPLICATION

(11) **EP 4 450 648 A2**
(43) Date of publication of application: **23.10.2024**
(21) Application number: 24183205.4
(22) Date of filing: 13.12.2017
(51) Int. Cl.: C12Q 1/6886

(54) **DETERMINING A PHYSIOLOGICAL CONDITION IN AN INDIVIDUAL BY ANALYZING CELL-FREE DNA FRAGMENT ENDPOINTS IN A BIOLOGICAL SAMPLE**

(30) Priority: 13.12.2016 US 201662433737 P
(62) Divisional of application: 17881105.5
(71) Applicant: Bellwether Bio, Inc., Seattle, Washington 98115 (US)
(72) Inventor: SNYDER, Matthew William, Seattle, 98107 (US); SHENDURE, Jay, Seattle, 98115 (US)
(74) Representative: Carpmaels & Ransford LLP

(57) **Abstract**

A method for using cell-free DNA to diagnose certain conditions.

## Description

### 1. BACKGROUND OF THE INVENTION

Cell-free DNA (cfDNA) is present in the circulating plasma, urine, and other bodily fluids of humans. The cfDNA comprises both single- and double-stranded DNA fragments that are relatively short (overwhelmingly less than 200 base-pairs) and are normally at a low concentration (*e.g.* 1-100 ng/mL in plasma). In the circulating plasma of healthy individuals, cfDNA is believed to primarily derive from apoptosis of blood cells, *i.e.* normal cells of the hematopoietic lineage. However, in specific situations, other tissues can contribute substantially to the composition of cfDNA in bodily fluids such as circulating plasma. This fact has been exploited in recent years - in conjunction with the emergence of new technologies for highly cost-effective DNA sequencing - towards the development of novel clinical diagnostics in at least three areas.
1) Reproductive medicine: In pregnant women, a proportion of cfDNA in circulating plasma derives from fetal or placental cells containing the fetal genome (median 14%; range 2-60%, increasing with gestational age but highly variable between pregnancies [Pmid 15033315]). Screening for genetic abnormalities in the fetus such as chromosomal trisomies can be achieved by deep sequencing of DNA libraries derived from circulating plasma cfDNA of a pregnant mother, a mixture of cfDNA derived from the maternal and fetal genomes. For example, if the fetus has trisomy 21, one expects to observe an excess of reads mapping to chromosome 21 in sequencing of maternal plasma cfDNA. As this test has demonstrated major advantages with respect to sensitivity and specificity over other noninvasive screening tests, noninvasive aneuploidy screening based on analysis of circulating cell-free DNA is now routinely offered to women with high-risk pregnancies.
2) Cancer diagnostics: In cancer, a proportion of cfDNA in circulating plasma can derive from the tumor (with the % contribution from the tumor increasing with cancer stage but highly variable between individuals and cancer types). Cancer is caused by abnormal cells exhibiting uncontrolled proliferation secondary to mutations in their genomes. The observation of these mutations in circulating plasma cfDNA - a mixture of cfDNA derived from normal cells and cancer cells - has substantial promise to effectively serve as a "liquid biopsy" - for example, to non-invasively monitor for tumor recurrence. The types of mutations in a cancer genome that can be detected in this way include small mutations, *e.g.* a change to a single base-pair, as well as copy number alterations, *e.g.* copy gain or copy loss of one or more large regions or entire chromosomes.
3) Transplant medicine: After a transplant is performed, from a donor to a recipient, the major risk to the recipient is allograft rejection. A major clinical challenge is determining whether and the extent to which rejection is occurring, and the gold standard method for assessing rejection for many types of solid organ transplants involves invasive biopsy. Recently, the presence and abundance of circulating plasma cfDNA derived from the donor has been explored as a noninvasive marker for detecting and monitoring allograft rejection. For example, for female recipients of a solid organ transplant from a male donor, cfDNA derived from the Y chromosome unambiguously derives from the allograft and can be quantified. More generally, donor-specific genotypes - for example, determined by genome-wide genotyping of common variants or whole genome sequencing of both donor and recipient - can be exploited to differentiate between donor-derived and recipient-derived molecules when performing deep sequencing of cfDNA from circulating plasma or other bodily fluids of a transplant recipient.

There are several shared characteristics of the above-described clinical diagnostic tests. First, each test relies on sequencing of cfDNA, generally from circulating plasma but potentially from other bodily fluids. The sequencing is usually 'shotgun', but in some implementations is targeted to particular regions of the human genome. Second, the cfDNA that one is sequencing is anticipated to derive from 2+ cell populations bearing genomes that differ from one another with respect to primary nucleotide sequence and/or copy number representation of particular sequences (*e.g.* maternal genome vs. fetal genome; normal genome vs. cancer genome; transplant recipient genome vs. transplant donor genome). Third, the basis for each test is to either detect or monitor these genotypic differences between the 2+ cell populations that contribute to the composition of cfDNA (*e.g.* fetal trisomy 21; cancer-specific somatic point mutations or aneuploidies; transplant donor-specific genotypes).

Although it is the basis for their success, the reliance of these cfDNA tests on genotypic differences nonetheless represents a major limitation. First, for all of these tests, the overwhelming majority of cfDNA molecules correspond to regions of the human genome where the 2+ cell populations that one is trying to distinguish are identical at the sequence level. Consequently, in applications where one is quantifying cfDNA molecules that unambiguously derive from a specific cell population based on cell-type specific genotype(s), the vast majority of sequencing reads are uninformative. In applications where one is predicting the copy number content of one of the cell populations based on relative coverage of genomic regions (e.g. detecting trisomies from sequencing of maternal circulating cfDNA), a higher depth of sequencing coverage is required than if the origin of individual cfDNA molecules was knowable, or at least could be assigned non-uniform probabilities. Second, there are numerous pathologies wherein tissue damage or inflammatory processes are taking place and the tissue-of origin composition of cfDNA might be expected to be altered as a consequence. However, these cannot always be detected by focusing on the genotypic differences between the contributing cell populations, simply because their genomes are identical or nearly identical. These include, for example, myocardial infarction (acute damage to heart tissue) and autoimmune disease (chronic damage to diverse tissues). However, they potentially also include many of the conditions described above such as cancer. For example, it has been observed that there is a major increase in the concentration of circulating plasma cfDNA in cancer, possibly disproportionate to the contribution from the tumor itself. This suggests that other tissues (e.g. stromal, immune system) may be contributing to circulating plasma cfDNA during cancer. However, these cell types have essentially unmutated genomes compared to the tumor, and as such cannot be readily distinguished from the cell types that normally contribute to cfDNA (e.g. normal cells of the hematopoietic lineage) based on genotypic differences.

We previously identified that cfDNA molecules carry an epigenetic signature of their cell type of origin, as evidenced in aggregate by the genomic coordinates of the millions of enzymatic fragmentation events giving rise to cfDNA during cell death. We observed that enzymatic fragmentation is biased, preferentially occurring at specific genomic coordinates in healthy individuals. We further determined that these fragmentation coordinates differ predictably across physiological conditions, owing in part to proportional contributions from additional cell types in which different fragmentation biases are at play. We proposed that these preferentially observed coordinates are influenced by the position of DNA-binding or DNA-contacting proteins, including nucleosomes and transcription factors, the ensemble of which serves to package the DNA in the nucleus and confers protection from enzymatic cleavage to specific base-pairs of DNA. We then demonstrated that analysis of the set of fragmentation coordinates, absent other differences at the primary sequence level, is sufficient to identify cell type contributors to total cfDNA in at least some physiological conditions, including cancer.

Finally, we showed that these findings could inform clinical decision making in the context of one or more physiological conditions. We demonstrated that the anatomical origin of the primary tumor could be identified using the described methods in at least some human cancers, which could inform therapeutic interventions or other treatment options.

### 2. SUMMARY OF THE INVENTION

The present application provides methods for determining whether a biological sample from an individual does or does not contain evidence of a specific physiological condition, by analyzing cfDNA fragment endpoints present in the biological sample and comparing these endpoints, or a statistical transformation thereof, to one or more reference datasets comprised of fragment endpoints, or a statistical transformation thereof, discovered in healthy individuals or individuals with a specific physiological condition. In some embodiments, the physiological condition in either the individual from whom the biological sample is obtained, or in the individuals from whom the reference datasets are obtained, is a specific type of cancer. In other embodiments, this physiological condition is pregnancy.

Some embodiments are drawn to a method of identifying a condition in a subject, the method comprising:
isolating a plurality of cfDNA fragments from a biological sample from the subject;
sequencing at least a portion of the plurality of cfDNA fragments;
mapping a plurality of cfDNA fragments to a reference genome;
assigning to each of a plurality of genomic positions a first value corresponding to the number of cfDNA fragments having their leftmost endpoint at said position and a second value corresponding to the number of cfDNA fragment having their rightmost endpoint at said position;
comparing the first value and the second value from the subject to corresponding values in a reference dataset from one or more reference subjects; and
identifying a probability of the condition in the subject based on the correlation of the subject's first value and second value to the corresponding values.

In some embodiments, a plurality of genomic positions is assigned four values, a first value corresponding to the number of cfDNA fragments having their leftmost endpoint at said position and where the fragments were derived from the Watson strand, a second value corresponding to the number of cfDNA fragments having their rightmost endpoint at said position and where the fragments were derived from the Watson strand, a third value corresponding to the number of cfDNA fragments having their leftmost endpoint at said position and where the fragments were derived from the Crick strand, and a fourth value corresponding to the number of cfDNA fragments having their rightmost endpoint at said position and where the fragments were derived from the Crick strand. In some embodiments, at least one of the multiple values assigned to each of a plurality of genomic positions is/are a statistical transformation of the number of cfDNA fragment endpoints meeting the stated criteria.

In some embodiments, the method further comprises generating a report listing a plurality of scores comparing the values from the subject to corresponding values in a reference dataset from one or more reference subjects. In some embodiments, the method further comprises recommending treatment for an identified condition in the subject. In some embodiments, the method further comprises treating the identified condition in the subject. In some embodiments, the identified condition is a cancer. In some embodiments, the identified condition is pregnancy. In some embodiments, the identified condition is transplant acceptance or rejection. In some embodiments, the value assigned to each of a plurality of genomic positions is a statistical transformation of the number of cfDNA fragment endpoints observed at the position.

### 3. BRIEF DESCRIPTION OF THE FIGURES

**FIG. 1** depicts a schematic representation of the role of strand and fragment orientation in the analysis of fragment endpoint coordinates.
**FIG. 2** depicts counts of left and right endpoints of cell-free DNA fragments tallied at each position in the neighborhood of each of 16,537 CTCF binding sites on the Watson (or "plus") strand.
**FIG. 3** depicts counts of all endpoints, including both left and right endpoints, of cell-free DNA fragments tallied at each position in the neighborhood of each of 16,537 CTCF binding sites on the Watson (or "plus") strand.
**FIG. 4** depicts alignment of transcription starts sites such that the first transcribed base falls at the 0 position, fragment endpoints are summed across sites at each position relative to the transcription start sites.

### 4. DETAILED DESCRIPTION OF THE EMBODIMENTS

### 4.1 ISOLATION OF CFDNA

cfDNA may be isolated by any method known to one skilled in the art.

As an example, a plasma sample may be added to a micro centrifuge tube. The sample is then treated with Proteinase K. Binding buffer is added. The sample is then mixed by inverting the tube and vortexing. The sample may then be centrifuged. The sample is then washed and the cfDNA is then eluted and residual ethanol removed.

### 4.2 SEQUENCING

After purification of cfDNA from biological fluids, for example using standard techniques, the fragments are subjected to one or more enzymatic steps to create a sequencing library. These enzymatic steps may include one or more of 5' phosphorylation, end repair with a polymerase, A-tailing with a polymerase, ligation of one or more sequencing adapters with a ligase, and linear or exponential amplification of a plurality of fragments with a polymerase. In some embodiments, a plurality of fragments whose sequence composition matches a pre-defined panel of sequences may be targeted or selected by hybridization-capture, such that a subset of the starting library is carried forward for additional steps.

Any method of sequencing known to one skilled in art can be used. For example, in some embodiments, sequencing is done by at least one of (i) the Sanger method or a variant of the Sanger method; (ii) the Maxam and Gilbert method or another chemical variant of the Maxam and Gilbert method; (iii) the Pyrosequencing method or a variant of the Pyrosequencing method; (iv) single molecule sequencing with an exonuclease or a variant of single molecule sequencing with an exonuclease; (v) electronic sequencing; and/or (vi) atomic sequencing. In some embodiments, sequencing is performed with 454 technology, the Illumina (Solexa) GA/Hi Seq/Mi Seq/Next Seq method, Applied Biosystems SOLiD method, the Ion Torrent method, and/or PacBio RS method. Any other method known to one skilled in the art may be used.

The biological sample may be any solid or liquid sample. In some embodiments, the biological sample is a solid sample. In some embodiments, the biological sample is a liquid sample such as a biological fluid. In some embodiments, the biological fluid from which cfDNA is purified is peripheral blood. In other embodiments, this biological fluid is plasma. In other embodiments, this biological fluid is urine. In other embodiments, this biological fluid is cerebrospinal fluid.

Following the construction of a sequencing library, the library is sequenced, for example using standard techniques, to generate a dataset consisting of at least one "read" (the ordered list of nucleotides comprising each sequenced molecule).

### 4.3 MAPPING

After sequencing of cfDNA and appropriate quality control of the resulting reads, these reads are mapped to a reference genome. The process of mapping identifies the genomic origin of each fragment on the basis of a sequence comparison - determining, for example, that a given fragment of cfDNA was originally part of a specific region of chromosome 12.

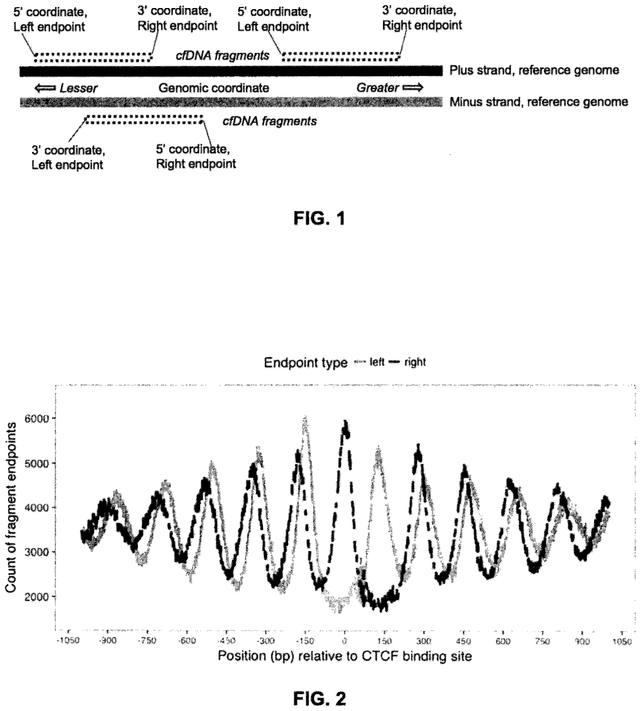

The procedure of mapping provides two endpoints for each mapped fragment. These endpoints are given numerical values ("coordinates"), representing the specific offset, relative to one end of a chromosome, of the fragment's location within the reference genome. These endpoints are further oriented in two dimensions, such that for every mapped fragment, a given endpoint's coordinate is either greater than or less than its partner's coordinate - in other words, is the left-most or right-most coordinate of the pair in two-dimensional space. This procedure produces, for each fragment, two endpoints: a left endpoint and a right endpoint. For all fragments, the left endpoint is the endpoint of the fragment having the lesser genomic coordinate, and the right endpoint is the endpoint of the fragment having the greater genomic coordinate (*see* FIG. 1). In some embodiments, all fragments from the minus strand are converted computationally to the plus strand by reverse-complementing the sequence and swapping the two endpoints.

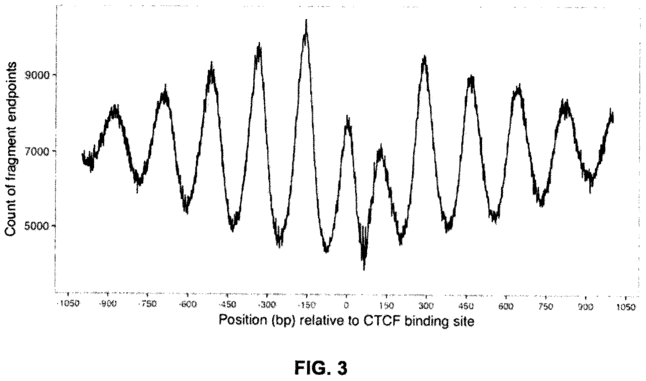

In some embodiments, the first value and the second value are considered separate values, each value corresponding to a leftmost endpoint and rightmost endpoint, respectively. In some embodiments, the left and right endpoints of the sequenced fragments are analyzed separately (*see,* FIG. 2). Treating left and right endpoints as distinct sets reveals the limits, in genomic space, of the region of protection from degradation conferred by the association of a DNA-binding protein, for example a transcription factor or a nucleosome, with the DNA itself. In some embodiments, the first value and second value are considered as a set of values. In some embodiments, the left and right endpoints of the sequenced fragments are pooled together for analysis (*see,* FIG. 3). Jointly considering all endpoints obscures information about the precise extent of the protected region of DNA, but continues to reveal the spacing between adjacent proteins.

In some embodiments, only fragments of a specific length are selected. In some embodiments, only fragments of about 100 base-pairs, fragments of about 110 base-pairs, fragments of about 120 base-pairs, fragments of about 130 base-pairs, fragments of about 140 base-pairs, fragments of about 150 base-pairs, fragments of about 160 base-pairs, fragments of about 170 base-pairs, fragments of about 180 base-pairs, fragments of about 190 base-pairs, or fragments of about 200 base-pairs are selected. In some embodiments, only fragments of a specific range of lengths are selected. In some embodiments, only fragments of a specific range with a lower limit of about 100 base-pairs, of about 110 base-pairs, of about 120 base-pairs, of about 130 base-pairs, of about 140 base-pairs, of about 150 base-pairs, of about 160 base-pairs, of about 170 base-pairs, of about 180 base-pairs, or of about 190 base-pairs are selected. In some embodiments, only fragments of a specific range with an upper limit of about 110 base-pairs, of about 120 base-pairs, of about 130 base-pairs, of about 140 base-pairs, of about 150 base-pairs, of about 160 base-pairs, of about 170 base-pairs, of about 180 base-pairs, of about 190 base-pairs, or of about 200 base-pairs are selected. In some embodiments, only fragments with lengths between 120 and 180 base-pairs selected.

In some embodiments, a plurality of these endpoints are classified based upon the strand, for example Watson or Crick, from which their associated, sequenced cfDNA fragment was derived. As used herein, "Watson" and "Crick" strands shall mean the following. A genomic reference is used to define two unequal arms of a genomic region. The Watson strand is the strand of the genomic region that has its 5'-end at the short end of the region and its 3'-end at the long end of the region. The Crick strand is the strand that has its 5'-end at the long end of the region and its 3'-end at the short end of the region. The Watson strand is often stored as the reference (+) strand in a genomic database. If no genomic reference is possible, then the Watson strand may be used as the reference strand and the Crick strand may be used as the complement.

FIG. 1 depicts a schematic representation of the role of strand and fragment orientation in the analysis of fragment endpoint coordinates. The reference genome consists of a plus strand (dark gray rectangle) and its reverse complement, the minus strand (light gray rectangle). Sequenced cfDNA fragments (dashed boxes) may be mapped to either strand. Each mapped fragment has one 5' endpoint coordinate and one 3' endpoint coordinate; the lesser of these two coordinates in genomic space is labeled the left endpoint and the greater of these two coordinates in genomic space is labeled the right endpoint. Fragments mapped to the plus strand have their 5' coordinates as their left endpoints and their 3' coordinates as their right endpoints; fragments mapped to the minus strand have their 3' coordinates as their left endpoints and their 5' coordinates as their right endpoints.

As used herein, "reference genome" shall refer to a nucleic acid sequence database, assembled as a representative example of a species' set of genes. Reference genomes are often assembled from the sequencing of DNA from a number of donors, so reference genomes do not accurately represent the set of genes of any single individual. Instead a reference genome provides a haploid mosaic of different DNA sequences from each donor. For example, without limitation, *GRCh37,* the Genome Reference Consortium human genome (build 37) is derived from thirteen anonymous volunteers from Buffalo, New York. For much of a genome, the reference provides a good approximation of the DNA of any single individual. For regions where there is known to be large scale variation, sets of alternate loci may be assembled alongside the reference locus.

The procedure of sample acquisition, library construction, DNA sequencing, and endpoint coordinate determination is applied to at least one biological sample derived from at least one healthy individual, resulting in a map of a plurality endpoints observed in a given sample. In some embodiments, at least 20%, 30%, 40%, 50%, 60%, 70%, 80%, or 90% of observed endpoints are mapped. In some embodiments, only a subset of the human reference genome is analyzed, resulting in a partial map restricted to this subset. In some embodiments, coordinates are tallied in binary fashion, resulting in a map in which each possible position takes on only a value of 1 or 0, representing observed or unobserved endpoints. In other embodiments, each possible position is assigned a numerical value determined by the number of endpoints observed at this position in a biological sample. In some embodiments, this numerical value is determined by a statistical transformation of the number of endpoints observed at this position. In one embodiment, this numerical value is determined by the probability that a randomly sampled fragment from a different biological sample will have one endpoint at a given coordinate.

In some embodiments, the sequence data defines positions of cfDNA fragment endpoints within a reference genome, for example, if the reference dataset is generated by sequencing of cfDNA from subject(s) with known disease. In some embodiments, the sequence data defines positions of cfDNA fragment endpoints within a reference genome for a leftmost endpoint and a rightmost endpoint.

These numerical values are calculated for a plurality of positions in the reference genome. The resulting positional numerical values are combined (e.g., by addition) for each biological sample to create a, or more than one, "reference dataset," where each reference dataset consists of at least one numerical score assigned to at least one genomic coordinate as determined from at least one biological sample derived from at least one individual with at least one given physiological condition.

As used herein, the term "reference dataset" refers to any type or form of data which can be correlated or compared to an attribute of the cfDNA in the subject's biological sample as a function of the coordinate within the genome to which cfDNA sequences are aligned. The reference dataset may be correlated or compared to an attribute of the cfDNA in the subject's biological sample by any suitable means. For example and without limitation, the correlation or comparison may be accomplished by analyzing frequencies of cfDNA endpoints, either directly or after performing a mathematical transformation on their distribution across windows within the reference genome, in the subject's biological sample in view of numerical values or any other states defined for equivalent coordinates of the reference genome by the reference dataset. In some embodiments, the reference dataset comprises a mathematical transformation of the cfDNA fragment endpoint probabilities, or a quantity that correlates with such probabilities. In some embodiments, the reference dataset comprises cfDNA fragment endpoint probabilities, or a quantity that correlates with such probabilities, for at least a portion of the reference genome associated with a particular physiological condition.

The reference dataset(s) may be sourced or derived from any suitable data source including, for example, public databases of genomic information, published data, or data generated for a specific population of reference subjects which may each have a common attribute (e.g., disease status). In some embodiments, the reference dataset comprises an RNA expression dataset. In some embodiments, the reference dataset comprises data that is generated from at least one tissue or cell-type that is associated with at least one physiological condition from the one or more reference subjects. In some embodiments, the reference dataset is generated by sequencing of cfDNA fragments from a biological sample from one or more individuals with a known physiological condition. In some embodiments, the reference dataset is generated from at least one healthy subject. In some embodiments, the reference dataset is generated from a biopsy from a tumor. In some embodiments, the biological sample from which the reference dataset is generated is collected from an animal to which human cells or tissues have been xenografted.

### 4.4 COMPARING

The step of comparing the values from the subject to corresponding values in the reference dataset from the one or more reference subjects may be accomplished in a variety of ways. In some embodiments, the values from the subject are compared to more than one reference dataset. In some embodiments, conditions associated with the reference datasets which correlate most highly with the values from the subject are deemed to be contributing. For example, and without limitation, if the cfDNA data includes a list of likely cfDNA endpoints and their locations within the reference genome, the reference datasets having the most similar list of cfDNA endpoints and their locations within the reference genome may be deemed to be contributing. As another non-limiting example, the reference datasets having the most correlation (or increased correlation, relative to cfDNA from a healthy subject) with a mathematical transformation of the distribution of cfDNA fragment endpoints from the biological sample may be deemed to be contributing.

### 4.5 CONDITIONS

The condition can be, for example, healthy, cancer, or pregnancy. In some embodiments, the condition can be a reproductive abnormality, a cancer, and/or a transplant rejection. In some embodiments, the at least one physiological condition is selected from the group consisting of: cancer, normal pregnancy, a complication of pregnancy (e.g., aneuploid pregnancy), myocardial infarction, systemic autoimmune disease, localized autoimmune disease, inflammatory bowel disease, allotransplantation with rejection, allotransplantation without rejection, stroke, and localized tissue damage.

### 4.6 FURTHER CHARACTERISTICS

In some embodiments, genomic coordinates in a reference dataset are further ranked by their assigned numerical values, and only those positions with a rank above or below a specific threshold are retained for further analysis. In some embodiments, the lowest 5%, 10%, 20%, 30%, 40%, 50%, 60%, 70%, 80%, or 90% of all positions are retained. In some embodiments, the highest 5%, 10%, 20%, 30%, 40%, 50%, 60%, 70%, 80%, or 90% of all positions are retained.

A new biological sample is obtained from an individual with an unknown physiological condition (or lack thereof). This sample is processed as described above, resulting in a new set of fragment endpoints. In some embodiments, a plurality of resulting fragment endpoints are retained for further analysis; in some embodiments, a subset of endpoints of fixed or proportional size is retained (*e.g*., 20%, 30%, 40%, 50%, 60%, 70%, 80%, or 90% of all endpoints, or 10 million total endpoints). This set of endpoints is compared to one or more reference datasets to calculate a separate numerical score for each comparison. In some embodiments, this score is determined by calculating the number or proportion of overlapping endpoints coordinates between the reference dataset and the new sample. In other embodiments, the score is determined by combining (*e.g.,* by multiplication or other mathematical operations) the numerical scores in the reference dataset for each overlapping endpoint, optionally by weighting the calculation on the basis of the number or proportion of endpoints observed at each coordinate in the biological sample.

In some embodiments, the method further comprises determining a score for each of at least some coordinates of the reference genome, wherein the score is determined as a function of at least the plurality of cfDNA fragment endpoints and their genomic locations, and wherein the step of comparing the value from the subject to corresponding values in a reference dataset from one or more reference subjects comprises comparing the scores to one or more reference dataset. The score may be any metric (e.g., a numerical ranking or probability) which may be used to assign relative or absolute values to a coordinate of the reference genome. For example, the score may consist of, or be related to a probability, such as a probability that the coordinate represents a location of a cfDNA fragment endpoint or a first value or a second value. Such scores may be assigned to the coordinate by any suitable means including, for example, by counting absolute or relative events (e.g., the number of cfDNA fragment endpoints) associated with that particular coordinate, or performing a mathematical transformation on the values of such counts in the region or a genomic coordinate. In some embodiments, the score for a coordinate is related to the probability that the coordinate is a location of a cfDNA fragment endpoint. In some embodiments, the reference data set comprises a mathematical transformation of the scores, such as a Fourier transformation of the scores. After calculating a numerical score for each comparison, a determination of physiological condition in the biological sample is made on the basis of comparing this numerical score to one or more scores derived from additional biological samples compared to the same reference dataset in the same manner. In one embodiment, one or more samples from individuals known to be healthy are compared to the reference dataset of healthy individuals in this manner, and the distribution of scores from such comparisons is determined. A new sample from an individual with unknown health status is separately compared in this manner, and the resulting score is compared to the observed distribution of scores, such that the determination of the physiological condition is a function of the comparison of a score to a threshold (*e.g.,* the bottom 5% of all scores). In some embodiments, a numerical confidence score is attached to each determination in each sample. In some embodiments, this numerical confidence score is the estimated probability that the individual from whom the biological sample was obtained truly has the physiological condition under study.

n some embodiments, the scores are associated with at least one orthogonal biological feature. In some embodiments, the orthogonal biological feature is associated with highly expressed genes. In some embodiments, the orthogonal biological feature is associated with lowly expressed genes. In some embodiments, at least some of the plurality of the scores has a value above a threshold (minimum) value. In such embodiments, scores falling below the threshold (minimum) value are excluded from the step of comparing the scores to a reference map.

In some embodiments, one or more scores are displayed or reported for each sample. In some embodiments, one or more scores with confidence intervals are displayed or reported for each sample. In some embodiments, one or more scores with confidence intervals are reported for each sample. In some embodiments, one or more conditions are identified in a display or report. In some embodiments, one or more conditions are identified in a display or report, along with one or more scores. In some embodiments, one or more conditions are identified in a display or report, along with one or more scores. In some embodiments, one or more therapies is recommended in a display or report. In some embodiments, a subject is treated for one or more conditions identified in the method.

### 5. EXAMPLES

### EXAMPLE 1: PLASMA SAMPLE

Human peripheral blood plasma from an anonymous male donor with a clinical diagnosis of stage IV small cell lung cancer was obtained from Conversant Bio (Huntsville, AL) and stored in 0.5 ml aliquots at -80°C until use.

### EXAMPLE 2: PURIFICATION OF DNA

Cell-free DNA was purified from 1.0 ml of plasma with the QiaAMP Circulating Nucleic Acids kit (Qiagen) as per the manufacturer's protocol. DNA was eluted in buffer AVE in 30 ul volume. A total DNA yield of 22.5 ng was quantified with a Qubit fluorometer (Invitrogen).

### EXAMPLE 3: PREPARING A SEQUENCING LIBRARY

Adapter 2 was prepared by combining 4.5 ul TE (pH 8), 0.5 ul 1M NaCl, 10 uL 500 uM oligo Adapter2.1 (CGACGCTCTTCCGATC/ddT/), and 10 ul 500 uM oligo Adapter2.2 (/5Phos/AGATCGGAAGAGCGTCGTGTAGGGAAAGAG*T*G*T*A), incubating at 95°C for 10 seconds, and ramping to 14°C at a rate of 0.1°C/s. Purified cfDNA fragments were dephosphorylated by combining 2X CircLigase II buffer (Epicentre), 5 mM MnCl2, and 1U FastAP (Thermo Fisher) with 0.5-10 ng fragments in 20 ul reaction volume and incubating at 37°C for 30 minutes. Fragments were then denatured by heating to 95°C for 3 minutes, and were immediately transferred to an ice bath. The reaction was supplemented with biotin- conjugated adapter oligo CL78 (/5Phos/AGATCGGAAG /iSpC3/iSpC3/iSpC3/iSpC3/iSpC3/iSpC3/iSpC3/ iSpC3/iSpC3/iSpC3/3BioTEG/) (5 pmol), 20% PEG-6000 (w/v), and 200U CircLigase II (Epicentre) for a total volume of 40 ul, and was incubated overnight with rotation at 60°C, heated to 95°C for 3 minutes, and placed in an ice bath. 20 ul MyOne C1 beads (Life Technologies) were twice washed in bead binding buffer (BBB) (10 mM Tris-HCl [pH 8], 1M NaCl, 1 mM EDTA [pH 8], 0.05% Tween-20, and 0.5% SDS), and resuspended in 250 ul BBB. Adapter-ligated fragments were bound to the beads by rotating for 60 minutes at room temperature. Beads were collected on a magnetic rack and the supernatant was discarded. Beads were washed once with 500 ul wash buffer A (WBA) (10 mM Tris-HCl [pH 8], 1 mM EDTA [pH 8], 0.05% Tween-20, 100 mM NaCl, 0.5% SDS) and once with 500 ul wash buffer B (WBB) (10 mM Tris-HCl [pH 8], 1 mM EDTA [pH 8], 0.05% Tween-20, 100 mM NaCl). Beads were combined with 1X Isothermal Amplification Buffer (NEB), 2.5 uM oligo CL9(GTGACTGGAGTTCAGACGTGTGCTCTTCCGATCT), 250 uM (each) dNTPs, and 24U Bst 2.0 DNA Polymerase (NEB) in a reaction volume of 50 ul, incubated with gentle shaking by ramping temperature from 15°C to 37°C at 1°C/minute, and held at 37°C for 10 minutes. After collection on a magnetic rack, beads were washed once with 200 ul WBA, resuspended in 200 ul of stringency wash buffer (SWB) (0.1X SSC, 0.1% SDS), and incubated at 45°C for 3 minutes. Beads were again collected and washed once with 200 ul WBB. Beads were then combined with 1X CutSmart Buffer (NEB), 0.025% Tween-20, 100 uM (each) dNTPs, and 5U T4 DNA Polymerase (NEB) and incubated with gentle shaking for 30 minutes at room temperature. Beads were washed once with each of WBA, SWB, and WBB as described above. Beads were then mixed with 1X CutSmart Buffer (NEB), 1mM ATP, 5% PEG-6000, 0.025% Tween-20, 2 uM double-stranded Adapter 2, and 10U T4 DNA Ligase (NEB), and incubated with gentle shaking for 2 hours at room temperature. Beads were washed once with each of WBA, SWB, and WBB as described above, and resuspended in 25 ul TET buffer (10 mM Tris-HCl [pH 8], 1 mM EDTA [pH 8], 0.05% Tween-20). Second strands were eluted from beads by heating to 95°C, collecting beads on a magnetic rack, and transferring the supernatant to a new tube. Library amplification was monitored by real-time PCR was terminated after 6 cycles.

### EXAMPLE 4: SEQUENCING AND PRIMARY DATA PROCESSING

The library was sequenced on both HiSeq 2000 and NextSeq 500 instruments (Illumina). A total of 908,512,803 fragments were sequenced, using read lengths of 2 x 50bp and 43/42 bp, depending on the sequencing run. Fragments shorter than or equal to the read length were consensus-called and adapter-trimmed. Remaining consensus single-end reads (SR) and the individual PE reads were aligned to the human reference genome (GRCh37, 1000 Genomes phase 2 technical reference,
ftp://ftp.1000genomes.ebi.ac.uk/vol1/ftp/technical/reference/phase2_
reference_assembly_sequence/) using the ALN algorithm in the software program *BWA* v0.7.10. PE reads were further processed with BWA SAMPE to resolve ambiguous placement of read pairs or to rescue missing alignments by a more sensitive alignment step around the location of one placed read end. Aligned SR and PE data were stored in BAM format using the *samtools* API. Individual BAM files for this sample were merged across lanes and sequencing runs to create one combined BAM file.

### EXAMPLE 5: FRAGMENT ENDPOINT EXTRACTION

Fragment endpoint coordinates were extracted from the combined BAM file with the *samtools* API and the *pysam* python library. Both outer alignment coordinates of PE data were extracted for properly paired reads. Both end coordinates of SR alignments were extracted when PE data was collapsed to SR data by adapter trimming. Left and right endpoints were separately tallied, where the left endpoint was defined as the numerically lowest genomic coordinate of the aligned fragment, and the right endpoint was defined as the numerically greatest genomic coordinate of the aligned fragment (i.e., by correcting for strand).

### EXAMPLE 6: ANALYSIS OF CTCF AND TRANSCRIPTION START SITES

Transcriptional repressor CTCF, also known as 11-zinc finger protein or CCCTC-binding factor, is a transcription factor that in humans is encoded by the *CTCF* gene. CTCF is involved in many cellular processes, including transcriptional regulation, insulator activity, V(D)J recombination and regulation of chromatin architecture. Genomic coordinates of CTCF sites were obtained by filtering motif predictions using the *fimo* software program (p-value cutoff 1e-04) with ChIP-seq peaks from ENCODE (TfbsClusteredV3 set, downloaded from http://hgdownload.cse.ucse.edu/goldenPath/hg19/endcodeDCC/ wgEncodeRegTfbsClustered). Only sites with binding motifs on the plus strand were retained. Genomic coordinates of transcription start sites were obtained from Ensembl Build version 75. Only protein-coding genes were used in this analysis, and sites were adjusted as needed for the direction of transcription.

### EXAMPLE 7: MAPPING CELL FREE DNA TO THE REFERENCE GENOME

Each sequenced cell-free DNA fragment is mapped to the reference genome with the software tools *bwa* or *bowtie* to obtain two genomic coordinates, one 5' and one 3' coordinate, determined by the genomic locations of the endpoints of the sequenced fragment. Not every fragment is mapped to the same strand as some fragments are mapped to the Watson (or "plus") strand, and other fragments will be mapped to the Crick (or "minus") strand. After mapping, the strand and orientation of each fragment is computationally determined with the *samtools* software program or *htslib* software toolkit. A fragment mapped to the minus strand has a 5' coordinate that is numerically lesser than its 3' coordinate, in genomic space *(see,* FIG. 1).

### EXAMPLE 8: TALLYING COUNTS OF LEFT AND RIGHT ENDPOINTS OF CELL-FREE DNA FRAGMENTS AT EACH POSITION IN THE NEIGHBORHOOD OF 16,537 CTCF BINDING SITES ON THE WATSON STRAND

Features were aggregated and aligned at starting coordinates while adjusting for strand and direction of transcription. Counts of left and right endpoints of cell-free DNA fragments are tallied at each position in the neighborhood of each of 16,537 CTCF binding sites on the Watson (or "plus") strand. Only fragments with lengths between 120 and 180 base-pairs are used. After aligning all CTCF binding sites such that the first base of the predicted binding motif falls at the 0 position, fragment endpoints are summed across sites at each position relative to the binding motif. Adjacent peaks of left endpoints *(see,* FIG.2, solid, light gray line) and right endpoints *(see* FIG. 2, dashed, dark gray line) are separated by an average of 147 base-pairs (bp). The genomic distance between adjacent left endpoint peaks is approximately 180 bp, indicating the distance between adjacent nucleosome dyads in native chromatin in the contributing cell types is approximately 180 bp *(see* FIG. 2).

### EXAMPLE 9: TALLYING COUNTS OF ALL ENDPOINTS, INCLUDING BOTH LEFT AND RIGHT ENDPOINTS, OF CELL-FREE DNA FRAGMENTS AT EACH POSITION IN THE NEIGHBORHOOD OF EACH OF 16,537 CTCF BINDING SITES ON THE WATSON STRAND

Counts of all endpoints, including both left and right endpoints, of cell-free DNA fragments are tallied at each position in the neighborhood of each of 16,537 CTCF binding sites on the Watson (or "plus") strand. Only fragments with lengths between 120 and 180 base-pairs are used. After aligning all CTCF binding sites such that the first base of the predicted binding motif falls at the 0 position, fragment endpoints are summed across sites at each position relative to the binding motif. Only the 180 bp spacing between adjacent peaks is observed; the finer resolution evidence of nucleosome positioning observed when separating left and right endpoints is lost in this analysis *(see* FIG. 3).

### EXAMPLE 10: TALLYING ENDPOINT COUNTS OF LEFT AND RIGHT ENDPOINTS OF CELL-FREE DNA FRAGMENTS AT EACH POSITION IN THE NEIGHBORHOOD OF 11,454 TRANSCRIPTION START SITES ON THE WATSON STRAND

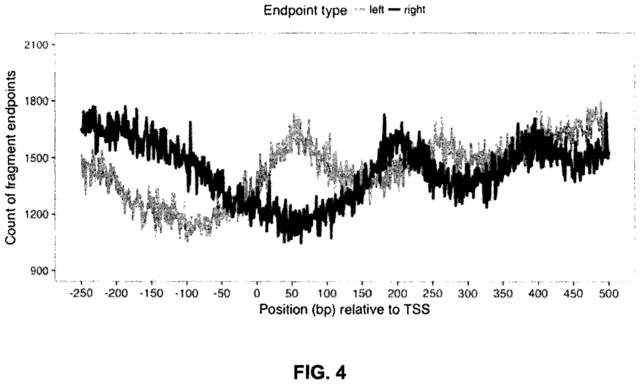

Transcription starts sites (TSSs) are the 5' genomic coordinates at which transcription of genes by RNA polymerase begins. Nucleosome spacing and positioning adjacent to TSSs is correlated with gene expression measurements, which vary across cell types and tissues in humans. TSSs have a hallmark nucleosome-free region (NFR) immediately upstream of the coordinate of the start site, adjacent to a strongly positioned "+1" nucleosome downstream of the start site. Counts of left and right endpoints of cell-free DNA fragments are tallied at each position in the neighborhood of each of 11,454 TSSs of annotated protein-coding genes on the Watson (or "plus") strand. Only fragments with lengths between 120 and 180 base-pairs are used. After aligning all TSSs such that the first transcribed base falls at the 0 position, fragment endpoints are summed across sites at each position relative to the TSS. The +1 nucleosome is evidenced by a peak of left endpoints (*see* FIG. 4, solid, light gray line), representing the nucleosome's 5' end, and a separate peak of right endpoints (*see* FIG. 4, dashed, dark gray line), representing the nucleosome's 3' end, at approximately 55 and 200 bp downstream of the TSS, respectively. The NFR is evidenced by an extended endpoint trough for both left and right endpoints, covering the TSS coordinate and extending upstream (see FIG. 4).

**The** invention is further characterised by the following numbered non-limiting embodiments:
1. A method of identifying a condition in a subject, the method comprising:
   isolating a plurality of cell free DNA (cfDNA) fragments from a biological sample from the subject;
   sequencing at least a portion of the plurality of cfDNA fragments;
   mapping a plurality of cfDNA fragments to a reference genome;
   assigning to each of a plurality of genomic positions a first value corresponding to the number of cfDNA fragments having their leftmost endpoint at said position and a second value corresponding to the number of cfDNA fragment having their rightmost endpoint at said position;
   comparing the first value and the second value from the subject to corresponding values in a reference dataset from one or more reference subjects; and
   identifying a probability of the condition in the subject based on the correlation of the subject's first value to the corresponding value in the reference dataset and/or the correlation of the subject's second value to the corresponding value in the reference dataset.
2. The method of embodiment 1 further comprising generating a report listing a plurality of scores comparing the values from the subject to corresponding values in a reference dataset from one or more reference subjects.
3. The method any of embodiments 1-2 further comprising recommending treatment for the identified condition in the subject.
4. The method of any of embodiments 1-3 further comprising treating the identified condition in the subject.
5. The method of any of embodiments 1-4 wherein the condition is a cancer.
6. The method of any of embodiments 1-3 wherein the condition is pregnancy.
7. The method of embodiment 1 where the value assigned to each of a plurality of genomic positions is a statistical transformation of the number of cfDNA fragment endpoints observed at the position.
8. The method of embodiment 1 where each of a plurality of genomic position receives two values, the first value corresponding to the number of cfDNA fragments having their leftmost endpoint at said position and where the fragments were derived from the Watson strand, the second value corresponding to the number of cfDNA fragments having their rightmost endpoint at said position and where the fragments were derived from the Watson strand.
9. The method of embodiment 1 where each of a plurality of genomic position receives four values, the first value corresponding to the number of cfDNA fragments having their leftmost endpoint at said position and where the fragments were derived from the Watson strand, the second value corresponding to the number of cfDNA fragments having their rightmost endpoint at said position and where the fragments were derived from the Watson strand, the third value corresponding to the number of cfDNA fragments having their leftmost endpoint at said position and where the fragments were derived from the Crick strand, and the fourth value corresponding to the number of cfDNA fragments having their rightmost endpoint at said position and where the fragments were derived from the Crick strand.
10. The method of any of embodiments 7-9 where at least one of the multiple values assigned to each of a plurality of genomic positions is/are a statistical transformation of the number of cfDNA fragment endpoints meeting the stated criteria.

## Claims

1. A method of identifying a condition in a subject, the method comprising:
isolating a plurality of cell free DNA (cfDNA) fragments from a biological sample from the subject;
sequencing at least a portion of the plurality of cfDNA fragments;
mapping a plurality of cfDNA fragments to a reference genome;
assigning to each of a plurality of genomic positions a first value corresponding to the number of cfDNA fragments having their leftmost endpoint at said position and a second value corresponding to the number of cfDNA fragment having their rightmost endpoint at said position;
comparing the first value and the second value from the subject to corresponding values in a reference dataset from one or more reference subjects; and
identifying a probability of the condition in the subject based on the correlation of the subject's first value to the corresponding value in the reference dataset and/or the correlation of the subject's second value to the corresponding value in the reference dataset.

2. The method of claim 1 further comprising generating a report which (a) lists a plurality of scores comparing the values from the subject to corresponding values in a reference dataset from one or more reference subjects, and/or (b) identifies one or more conditions.

3. The method any of claims 1-2 further comprising recommending treatment for the identified condition in the subject, for example wherein one or more therapies are recommended in a report.

4. The method of any of claims 1-3 further comprising treating the identified condition in the subject.

5. The method of any of claims 1-4 wherein the condition is a cancer.

6. The method of any of claims 1-3 wherein the condition is pregnancy.

7. The method of claim 1 where the value assigned to each of a plurality of genomic positions is a statistical transformation of the number of cfDNA fragment endpoints observed at the position.

8. The method of claim 1 where each of a plurality of genomic position receives two values, the first value corresponding to the number of cfDNA fragments having their leftmost endpoint at said position and where the fragments were derived from the Watson strand, the second value corresponding to the number of cfDNA fragments having their rightmost endpoint at said position and where the fragments were derived from the Watson strand.

9. The method of claim 1 where each of a plurality of genomic position receives four values, the first value corresponding to the number of cfDNA fragments having their leftmost endpoint at said position and where the fragments were derived from the Watson strand, the second value corresponding to the number of cfDNA fragments having their rightmost endpoint at said position and where the fragments were derived from the Watson strand, the third value corresponding to the number of cfDNA fragments having their leftmost endpoint at said position and where the fragments were derived from the Crick strand, and the fourth value corresponding to the number of cfDNA fragments having their rightmost endpoint at said position and where the fragments were derived from the Crick strand.

10. The method of any of claims 7-9 where at least one of the multiple values assigned to each of a plurality of genomic positions is/are a statistical transformation of the number of cfDNA fragment endpoints meeting the stated criteria.
